# EUROPÄISCHE PATENTANMELDUNG

(11) **EP 4 186 435 A1**
(43) Veröffentlichungstag der Anmeldung: **31.05.2023**
(21) Anmeldenummer: 21210851.8
(22) Anmeldetag: 26.11.2021
(51) Int. Cl.: A61B 6/14, A61B 6/00

(54) **GERÄT UND VERFAHREN ZUR ERSTELLUNG DENTALER RÖNTGENAUFNAHMEN MIT HÖHEN-SPEZIFISCHER DOSISAPPLIKATION**

(71) Anmelder: Sirona Dental Systems GmbH, 64625 Bensheim (DE); DENTSPLY SIRONA Inc., York, 17401-2991 (US)
(72) Erfinder: Maur, Susanne, 64625 Bensheim (DE); Eichner, Stefan, 64625 Bensheim (DE)
(74) Vertreter: Özer, Alpdeniz

(57) **Zusammenfassung**

Die Vorliegende Erfindung betrifft ein extraorales dentales Röntgengerät (2) für Aufnahmen eines Patienten im dentalen Bereich umfassend: ein (3), welches mindestens zwei Einzelstrahlern (3-1;3-2) jeweils zum Emittieren von Röntgenstrahlungen aufweist, die mindestens entlang einer vorbestimmten Richtung (z) versetzt sind; und einen Röntgendetektor (4) zum mindestens teilweisen Detektieren der von den Einzelstrahlern (3-1;3-2) emittierten Röntgenstrahlungen, wobei das Strahlerarray (3) und der Röntgendetektor (4) um eine zur der genannten vorbestimmten Richtung (z) parallel laufenden Achse (z') relativ zueinander beweglich gelagert sind; gekennzeichnet dadurch, dass es ferner eine Steuerungsvorrichtung zum separaten Ansteuern der Einzelstrahler (3-1;3-2) aufweist, wobei die Steuerungsvorrichtung so ausgebildet ist, dass die emittierten Röntgenstrahlungen der mindestens zwei Einzelstrahler (3-1;3-2) sich in der Intensität und/oder Spektralverteilung der Röntgenstrahlungen unterscheiden um eine entlang der vorbestimmten Richtung (z) variierbare Intensität und/oder Spektralverteilung zu bewirken.

## Beschreibung

### TECHNISCHES GEBIET DER ERFINDUNG

Die vorliegende Erfindung bezieht sich auf ein Röntgengerät für PAN, CEPH und/oder DVT im dentalen Bereich.

### HINTERGRUND DER ERFINDUNG

Extraorale dentale Röntgensysteme nutzen üblicherweise konstante Strahlparameter, sowohl pro Projektionsbild als meist auch während des Umlaufs. Aufgrund der Patientenanatomie führt dies in den Röntgenprojektionen zu Bildbereichen mit hohen und niedrigen Signalanteilen. Dadurch werden die Röntgendetektoren teilweise in einem ungünstigen Dosisbereich genutzt, so dass es zu Sättigungseffekten im Hochdosisbereich und Nicht-Linearitäten im Niedrigdosisbereich des Röntgendetektors kommt. Viele Röntgendetektoren sind nicht in der Lage, die gegebene Intensitätsdynamik ausreichend zu erfassen.

Allgemein entscheidet der Arzt nach seiner Erfahrung über die Strahlungsparameter, abhängig von dem Patienten und von der Indikation. Hierbei sollte das ALARA-Prinzip Anwendung finden. Einige extraorale dentale Röntgensysteme nutzen Scout-Aufnahmen zur Anpassung des Bildaufnahmebereichs an den Patienten. Scout-Aufnahmen können auch für eine initiale Dosis-Schätzung und Justierung genutzt werden. Scout-Aufnahmen stellen jedoch einen zusätzlichen Aufwand im Workflow der Aufnahme dar. Einige extraorale dentale Röntgensysteme passen die Strahlparameter während des Umlaufs an, um im Bereich der Wirbelsäule eine erhöhte Dosis zu applizieren. Allgemein wird bei Panorama-Aufnahmen (PAN) oft eine kV-Erhöhung im Bereich der Wirbelsäule angewendet.

### OFFENBARUNG DER ERFINDUNG

Den Erfindern ist derzeit keine Technik bekannt, die eine gezielte adaptive höhen-spezifische Anpassung der Strahlungsparameter bezüglich der Patienten-Anatomie ermöglicht. Die bekannten Techniken ermöglichen auch keine anatomisch bedingte höhen-spezifische Dosisapplikation während des Umlaufs. Eine anatomie-spezifische Dosisapplikation ist für gewöhnlich im Stand der Technik nicht möglich. Somit wird oft zu viel Dosis appliziert, da das höhen-spezifische, anatomie-bedingte Absorptionsverhalten des Patientenkopfes nicht berücksichtigt wird und die Wahl der Strahlungsparameter meist aufgrund der stark absorbierenden anatomischen Strukturen bestimmt wird.

Bei DVT- und PAN-Aufnahmen gibt es eine automatische Dosisanpassung an den Patienten durch eine Scout-Aufnahme oder eine Nachregelung der Strahlparameter, wobei diese jedoch *nicht* höhen-spezifisch ist.

Ziel der vorliegenden Erfindung ist ein Gerät und Verfahren zur Erstellung dentaler Röntgenaufnahmen (DVT, PAN und/oder CEPH) mit höhen-spezifischer Dosisapplikation bereitzustellen.

Dieses Ziel wurde durch das Röntgengerät nach Anspruch 1, und das Verfahren nach Anspruch 11 erreicht. Die Gegenstände der abhängigen Ansprüche beziehen sich auf bevorzugte Ausführungsformen bzw. Weiterentwicklungen.

Das erfindungsgemäße extraorale dentale Röntgengerät dient zur Aufnahme eines Patienten. Es umfasst ein Strahlerarray, welches mindestens zwei Einzelstrahlern jeweils zum Emittieren von Röntgenstrahlungen aufweist, die mindestens entlang einer vorbestimmten Richtung versetzt sind; und einen Röntgendetektor zum mindestens teilweisen Detektieren der von den Einzelstrahlern emittierten Röntgenstrahlungen, wobei das Strahlerarray und der Röntgendetektor um eine zur vorbestimmten Richtung parallel laufenden Achse relativ zueinander beweglich gelagert sind; wobei, dass es ferner eine Steuerungsvorrichtung zum separaten Steuern der Einzelstrahler aufweist, wobei die Steuerungsvorrichtung so ausgebildet ist, dass die emittierten Röntgenstrahlungen der mindestens zwei Einzelstrahler sich in der Intensität und/oder Spektralverteilung der Röntgenstrahlungen unterscheiden um eine entlang der vorbestimmten Richtung variierbare Intensität und/oder Spektralverteilung zu bewirken.

Die Vorteile der vorliegenden Erfindung sind eine Verbesserung der Bildqualität aufgrund der verbesserten Kontrastdarstellung im Hoch- und Niedrigdosisbereich, die sich durch die günstigere Verwendung des Dynamikbereiches des Röntgendetektors ergibt. Zudem kann die Gesamtdosis der Aufnahme reduziert werden, da in weniger stark absorbierenden anatomischen Bereichen oder weniger relevanten Bildbereichen die Dosis minimiert werden kann. Hierzu ist keine zusätzliche Aufnahme wie die Scout-Aufnahme erforderlich, was die Bedienung des Röntgengeräts vereinfacht und Zeit spart. Die anatomie-spezifische Dosis-Anpassung führt zu einer verlässlichen Bildqualität, da unterschiedliche Patientenpositionierungen und ungünstige Strukturüberlagerungen kompensiert werden. Dies führt zu weniger Widerholungsaufnahmen und somit weniger Dosisapplikation für den Patienten und eine vereinfachte Bedienung des Röntgengeräts für den Anwender.

Die anatomie-spezifische Dosisanpassung kann auch beispielsweise über sequenzielle oder zeitgleich angesteuerte Einzelstrahler, über die zusätzliche Nutzung einer oder mehrerer Blendenvorrichtungen und/oder Filtervorrichtungen realisiert werden. Zusätzlich können die Intensität und/oder Spektralverteilung der Röntgenstrahlungen während der Aufnahme in Abhängigkeit vom Signal des Röntgendetektors vorzugsweise aktiv nachgeregelt werden. Zusätzlich kann die Betriebsart des Röntgendetektors aktiv angepasst werden.

Die Aufnahmeparameter der Röntgenaufnahme sind beispielsweise vordefiniert oder durch den Benutzer selektiv konfigurierbar. Die Aufnahmeparameter umfassen das gewünschte Bildaufnahmeverfahren, den Zielwert für Intensität und/oder Spektralverteilung der Röntgenstrahlungen, die Kollimierung durch die Blendenvorrichtungen, die Größe und/oder Lage des Aufnahmebereichs im Patientenkopf, die Trajektorie des Strahlerarrays und des Röntgendetektors usw.

### KURZBESCHREIBUNG DER ZEICHNUNGEN

In der nachfolgenden Beschreibung wird die vorliegende Erfindung anhand von beispielhaften Ausführungsformen und unter Bezugnahme auf die Zeichnungen näher erläutert, wobei
Abb.1 - zeigt eine schematische Darstellung eines computergestützten DVT-Systems nach einer Ausführungsform;
Abb.2 - zeigt eine detaillierte schematische Teilansicht des extraoralen Röntgengerätes nach einer Ausführungsform.

Die in den Zeichnungen gezeigten Referenznummern bezeichnen die unten aufgeführten Elemente, auf die in der nachfolgenden Beschreibung der beispielhaften Ausführungsformen Bezug genommen wird.
- 1.: DVT-System
- 2.: Extraorales Röntgengerät
- 3.: Strahlerarray
- 3-1: Röntgen-Einzelstrahler
- 3-2: Röntgen-Einzelstrahler
- 4.: Röntgendetektor
- 5.: Bedienungseinheit
- 6.: Kopffixierung
- 7.: Aufbiss
- 8.: Rechner
- 9.: Anzeige
- 10.: Messobjekt bzw. Patientenkopf
- 11.: Kieferbogen
- 12.: Strahlkegel für Einzelstrahler
- 12'.: Strahlkegel für Einzelstrahler.
- s: Überlappungsbereich
- z: vorbestimmte Richtung
- z': Parallellaufende Achse

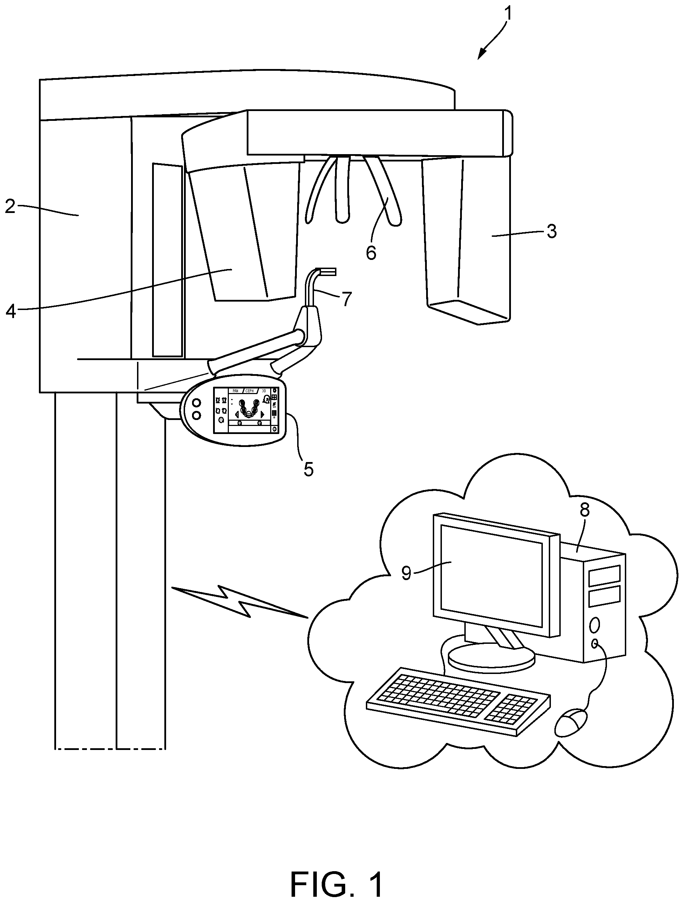

Abb. 1 zeigt ein Ausführungsbespiel für ein erfindungsgemäßes computergestütztes DVT-System (1). Das DVT-System (1) hat ein extraorales dentales Röntgengerät (2). Hierzu umfasst die vorliegende Erfindung auch ein Computerprogramm mit computerlesbarem Code. Das Computerprogramm kann auf einem Speichermedium bereitgestellt werden. Das extraorale dentale Röntgengerät (2) dient zur Durchführung der Patientenaufnahme, wobei die 2D Röntgenbilder bzw. das Sinogramm erzeugt wird. Hierzu hat das Röntgengerät (2) einen Röntgen Strahlerarray (3) und einen Röntgendetektor (4), die während der Aufnahme um den Patientenkopf mit einer konstanten bzw. variablen Geschwindigkeit gedreht werden. Das Strahlerarray (3) besteht aus mehreren kleinen, schnellschaltbaren Einzelstrahlern (3-1;3-2), die räumlich verteilt angeordnet und individuell steuerbar sind. Dies ermöglicht die Erstellung von Röntgenprojektionen aus unterschiedlichen Projektionswinkeln ohne Gerätebewegung. Die Einzelstrahler (3-1;3-2) des Strahlerarrays (3) können beispielsweise Kaltkathodenröhren z.B. aus Karbonnanoröhren umfassen. Das Röntgengerät (2) hat ein Drehmechanismus. Die Trajektorie des Strahlerarrays (3) und des Röntgendetektors (4) während der Aufnahme kann eine Kreisbahn beschreiben. Alternativ kann sie eine davon abweichende Form annehmen. Das Röntgen Strahlerarray (3) wird später detaillierter erklärt.

Das Röntgengerät (2) umfasst ferner eine Bedienungseinheit (5) um dasselbe zu bedienen bzw. zu konfigurieren. Wie in Abb. 1 gezeigt kann ein Patient mit dem Aufbiss (7) und der Kopffixierung (6) im Röntgengerät (1) positioniert werden. Das DVT-System (1) umfasst ferner, vorzugsweise einen Rechner (8) oder eine Recheneinheit, die mit dem Röntgengerät (2) verbunden werden kann, und vorzugsweise eine Anzeige (9), u.a. um die erzeugte Datensätze zu visualisieren. Der Aufbiss (7) und die Kopffixierung (6) dienen als Vorrichtung (6,7) zum Positionieren des Patienten. Der Rechner (8) kann über ein lokales Netzwerk (nicht gezeigt) oder alternativ über das Internet mit dem Röntgengerät (2) verbunden werden. Der Rechner (8) kann Teil einer Cloud sein. Die Berechnungen können in der Cloud stattfinden. Alternativ kann der Rechner (8) in das Röntgengerät (2) integriert werden. Der Rechner (8) führt das Computerprogramm aus und liefert die Datensätze, u.a. auch für die Visulisierung auf der Anzeige (9). Die Anzeige (9) kann räumlich von dem Röntgengerät (2) getrennt sein. Der Rechner (8) kann vorzugsweise auch das Röntgengerät (2) steuern. Alternativ können separate Rechner für die Steuerung und die Rekonstruktion benutzt werden.

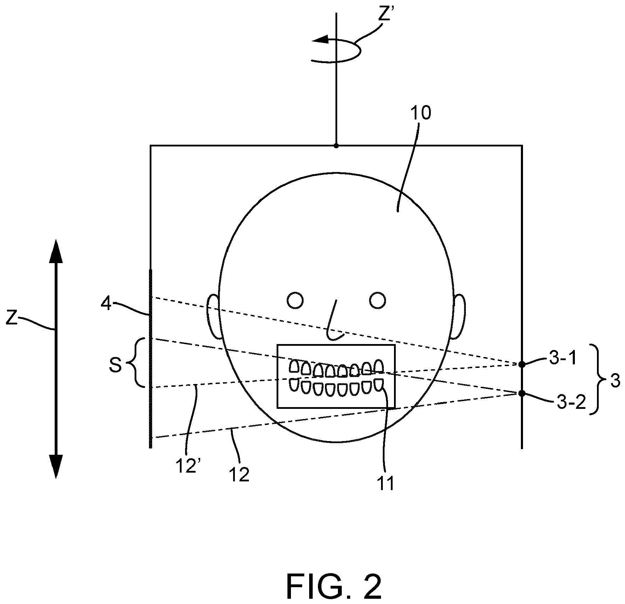

Abb.2 zeigt eine detaillierte schematische Teilansicht des Röntgengeräts (2). Wie in Abb.2 gezeigt, weist das Strahlerarray (3) mindestens zwei Einzelstrahler (3-1;3-2) jeweils zum Emittieren von Röntgenstrahlungen auf, die mindestens entlang einer vorbestimmten Richtung (z) (z.B. in der senkrechten Richtung) durch einen vorbestimmten Abstand versetzt sind. Das Röntgengerät (2) kann Aufrecht gestellt sein wobei der Patient stehend posititonert wird. Alternativ könnte Das Röntgengerät (2) horizantal oder vorzugsweise über einen Wnkelbereich verstellbar diagonal aufegstellt werden, wobei der Patient liegend oder sitzend mittels einer Rückenlehne posititonert wird. Der Abstand ist fest definiert kann aber auch vorzugsweise aktiv geregelt werden. Der Röntgendetektor (4) ist ausgebildet zum mindestens teilweisen Detektieren der von den Einzelstrahlern (3-1;3-2) emittierten Röntgenstrahlungen, wobei die Strahlkegel der Einzelstrahler (12; 12') auf dem Röntgendetektor (4) einen Überlappungsbereich (s) aufweisen können. Für PAN und DVT sind das Strahlerarray (3) und der Röntgendetektor (4) um eine zur vorbestimmten Richtung (z) parallellaufenden Achse (z') drehbar gelagert. Für one-shot-CEPH könnte der Röntgendetektor (4) festmontiert sein oder nicht gedreht werden. Für linear-CEPH ist der Röntgendetektor (4) verschiebbar gelagert. Alternativ kann ein zusätzlicher Röntgendetektor (nicht gezeigt) auf einem Ausleger Arm für CEPH verwendet werden. Das Röntgengerät (2) kann ein Kombigerät für PAN, DVT, und CEPH sein oder nur eine oder zwei der genannten Modalitäten unterstützen. Das Röntgengerät (1) weist eine Steuerungsvorrichtung *(nicht gezeigt)* zum separaten Steuern der Einzelstrahler (3-1;3-2) auf, wobei die Steuerungsvorrichtung so ausgebildet ist, dass die emittierten Röntgenstrahlungen der mindestens zwei Einzelstrahler (3-1;3-2) sich in der Intensität und/oder Spektralverteilung der Röntgenstrahlungen unterscheiden um eine entlang der vorbestimmten Richtung (z) variierbare Intensität und/oder Spektralverteilung zu bewirken. Das Ansteuern umfasst die separate Regelung des Stroms und/oder der Spannung zu dem jeweiligen Einzelstrahler (3-1;3-2). Dadurch kann eine höhen-spezifische Dosisapplikation aktiv ermöglicht werden.

In einer Ausführungsform wird die Intensität und/oder Spektralverteilung der Röntgenstrahlungen der Einzelstrahler während des Gerätebewegung variiert (z.B. durch Variation von Strom und/oder Spannung). Dies hat den Vorteil, dass Sättigungseffekte im Hochdosisbereich und Nicht-Linearitäten im Niedrigdosisbereich des Röntgendetektors in Abhängigkeit vom Projektionswinkel verringert werden können.

In einer vorteilhaften Ausführungsform ist die Steuerungsvorrichtung so ausgebildet, dass die Einzelstrahler (3-1;3-2) *sequenziell oder zeitgleich* angesteuert werden können, und die detektierten Signale des Röntgendetektors (4) synchron zur Ansteuerung der Einzelstrahler (3-1;3-2) ausgelesen werden. Bei der sequenziellen Ansteuerung der Einzelstrahler können die Signale des Röntgendetektors für jeden Einzelstrahler separat ausgelesen werden und somit Mehrdeutigkeiten im Überlappungsbereich (s) in der Rekonstruktion vermieden werden. Bei der zeitgleichen Ansteuerung der Einzelstrahler können die Signale des Röntgendetektors für mehrere Einzelstrahler zeitgleich ausgelesen werden. Somit kann eine geringere Ausleserate des Röntgendetektors genutzt werden, was eine längere Integrationszeit der Röntgenstrahlungen ermöglicht und Nicht-Linearitäten im NiedrigDosisbereich verringert.

In einer vorteilhaften Ausführungsform weisen die Einzelstrahler (3-1;3-2) jeweils eine eigene Blendenvorrichtung zur Kollimierung der Röntgenstrahlung auf. Dies hat den Vorteil, dass die Strahlkegel der Einzelstrahler (12; 12') besser voneinander abgegrenzt werden können und der Überlappungsbereich (s) der Einzelstrahler minimiert und besser definiert werden kann. Alternativ oder zusätzlich weist das Strahlerarray (3) eine gemeinsame Blendenvorrichtung auf. Die Blendenvorrichtung kann durch eine Festblende oder eine mechanisch motorisch veränderbare Blende realisiert werden. Die Blendenvorrichtung hat die Vorteile, dass die Strahlenbelastung für den Patient minimiert wird, die Streustrahlung reduziert wird und die aktive nutzbare Fläche des Röntgendetektors nicht überstrahlt wird.

In einer weiteren vorteilhaften Ausführungsform weisen die Einzelstrahler (3-1;3-2) jeweils eine eigene Filtervorrichtung auf. Diese Filtervorrichtung dient der Vorfilterung, bzw. Abschwächung, der Röntgenstrahlungen und der Veränderung des Röntgenspektrums. Bei der Filtervorrichtung handelt sich um einen fest installierten Filter oder eine mechanisch motorisch veränderbare Filtervorrichtung wie zum Beispiel ein Filterrad mit unterschiedlichen Filtermaterialien. Die Filtervorrichtung umfasst zum Beispiel Filtermaterialien wie Kupfer oder Aluminium. Die Filtervorrichtung ermöglicht beispielsweise eine Aufhärtung der Röntgenstrahlungen, was zu einer besseren Darstellung der Knochenstrukturen führt. Außerdem wird der Anteil der weichen Röntgenstrahlung im Röntgenspektrum reduziert, welches wenig zur Darstellung der Knochen- und Zahnstrukturen beiträgt, aber für den Patienten eine schädliche Strahlenbelastung darstellt. Weist jeder Einzelstrahler (3-1;3-2) jeweils eine eigene Filtervorrichtung auf, kann zum Beispiel die Filtervorrichtung an die Intensität und/oder Spektralverteilung der Röntgenstrahlungen der Einzelstrahler angepasst werden. Alternativ weist das Strahlerarray (3) eine gemeinsame Filtervorrichtung auf, welche die Röntgenstrahler aller Einzelstrahler gemeinsam filtert, unabhängig von der Intensität und angepassten Spektralverteilung der Einzelstrahler. Somit kann zum Bespiel eine allgemeine Strahlaufhärtung realisiert werden.

In einer weiteren vorteilhaften Ausführungsform ist die Betriebsweise des Röntgengeräts (2) vom Benutzer mittels einer Eingabevorrichtung bzw. Bedieneinheit (5) bzgl. mindestens einer der folgenden Konfigurationsgrößen konfigurierbar:
- Bildaufnahmeverfahren, wie zum Beispiel PAN, CEPH oder DVT
- Zielwert für Intensität und/oder Spektralverteilung der Röntgenstrahlungen, welche bei der Variation berücksichtigt werden, zum Beispiel als Zielwert für einen Maximalwert oder einen Mittelwert,
- Kollimierung durch die Blendenvorrichtungen, zum Beispiel auf den Ober- oder den Unterkiefer-Bereich,
- Größe und/oder Lage des Aufnahmebereichs im Patientenkopf, zum Beispiel zur Beschränkung auf die linke oder rechte Seite der Kiefer oder ausgewählte anatomische Strukturen,
- Trajektorie des Strahlerarrays (3) und des Röntgendetektors (4), zum Beispiel zur Reduktion von Bildartefakten,
wobei die Steuerungsvorrichtung weiter ausgebildet ist die Einzelstrahler auf Basis der Benutzerkonfiguration zu steuern.

In einer weiteren vorteilhaften Ausführungsform ist die Steuerungsvorrichtung weiter ausgebildet die Einzelstrahler für die Aufnahme so zu steuern, dass die Intensität und/oder Spektralverteilung der Röntgenstrahlungen adaptiv an die Anatomie des Patienten angepasst werden. Dies hat den Vorteil, dass Sättigungseffekte im Hochdosisbereich und Nicht-Linearitäten im Niedrigdosisbereich des Röntgendetektors verringert werden. Außerdem kann in Dosis-sensiblen anatomischen Bereichen, wie zum Beispiel den Augenhöhlen, die Dosisbelastung für den Patienten reduziert werden. Zudem können bei stark absorbierenden anatomischen Strukturen, wie zum Beispiel der knöchernen oder metallenen Strukturen, eine erhöhte Intensität und/oder angepasste Spektralverteilung appliziert werden, um Nicht-Linearitäten des Röntgendetektors in den Bildbereichen mit starker Absorption zu vermeiden. Wobei die Steuerungsvorrichtung die Anatomie des Patienten nach mindestens einer der folgenden Varianten ermittelt:
(a) basierend auf allgemeinen Annahmen zur menschlichen Anatomie wie zum Beispiel empirischer biometrischer Daten,
(b) basierend auf der Einstellung einer Vorrichtung zum Positionieren des Patienten, z.B. kann über die Position eines Aufbisses oder einer Schläfenstütze die Größe des Patienten oder die Dicke des Patientenschädels abgeleitet werden,
(c) basierend auf der vorbekannten Anatomie des Patienten, wie zum Beispiel mittels einer Scout-Aufnahme, eines optischen Oberflächenmodells, oder eines anderen vorhandenen Bild-Datensatzes (PAN, CEPH, 3D) des gleichen Patienten. Die Position, bzw. die Höhe, des Strahlerarrays und des Röntgendetektors ist vorzugsweise verstellbar und dem Gerät bekannt. Das Wissen über die vorbekannte Anatomie des spezifischen Patienten kann in einer Datenbank hinterlegt werden bzw. aus der Datenbank abgerufen werden.

In einer weiteren vorteilhaften Ausführungsform ist die Steuerungsvorrichtung weiter ausgebildet die Einzelstrahler für die Aufnahme so zu steuern, dass die Intensität und/oder Spektralverteilung der Röntgenstrahlungen während der Aufnahme in Abhängigkeit vom Signal des Röntgendetektors nachgeregelt werden. Dies hat das Ziel, zu niedrige und zu hohe Signale am Röntgendetektor zu vermeiden, um Sättigungseffekte im Hochdosisbereich und Nicht-Linearitäten im Niedrigdosisbereich des Röntgendetektors zu reduzieren und den Röntgendetektor in einem möglichst linearen Arbeitsbereich zu betreiben.

In einer weiteren Ausführungsform wird die Drehgeschwindigkeit des Röntgendetektors (4) und des Strahlerarrays (3) während der Aufnahme variiert. Bei gleichbleibender Intensität und Spektralverteilung kann durch eine verringerte Drehgeschwindigkeit die gesamte angewandte Dosis erhöht werden oder umgekehrt. Dadurch kann die auf eine jeweilige lokale Struktur angewandte Dosis zusätzlich zu den voran beschriebenen Möglichkeiten gesteuert werden.

In einer weiteren vorteilhaften Ausführungsform ist die Steuerungsvorrichtung weiter ausgebildet mindestens einen Einzelstrahler während der Aufnahme so zu steuern, dass die Intensität und/oder Spektralverteilung der Röntgenstrahlung des Einzelstrahlers nach einer vorgegebenen Sequenz variiert werden, welche eine Frequenz von mindestens 50 Hz aufweist. Die Amplitude kann von der Patientenanatomie oder dem gemessenen Signal am Röntgendetektor abhängen. Die Rekonstruktionssoftware verrechnet die Einzelprojektionen der Einzelstrahler derart, dass sowohl im Niedrig- als auch im Hochdosisbereich Strukturen gut abgebildet werden. Das ermöglicht Aufnahmen mit hohem Kontrast, besonders bei Röntgendetektoren mit kleinem dynamischem Bereich, bzw. geringer Bit-Tiefe. Dieses Verfahren ist analog zu einer HDR-Aufnahme in der digitalen Fotographie.

In einer weiteren vorteilhaften Ausführungsform wird die Betriebsart des Röntgendetektors für die jeweils verwendeten Intensität und/oder Spektralverteilung der Röntgenstrahlungen komplett oder bereichsweise vor oder während der Aufnahme angepasst, zum Beispiel in seiner Betriebsart bezüglich des Dynamikbereichs, der Ausleserate, des ausgelesenen Bereichs und/oder der Verstärkung.

In einer weiteren vorteilhaften Ausführungsform weist das extraorale Röntgengerät (2) eines oder mehrere der PAN-, CEPH-, DVT- Bildaufnahmeverfahren auf, wobei diese durch den Benutzer wählbar sind.

Nach dem erfindungsgemäßen Verfahren wird ein gemeinsames Bild für PAN/CEPH oder ein Volumen für DVT aus Signalen des Röntgendetektors (4) des extraoralen dentalen Röntgengeräts (2) berechnet, wobei die Information der Ansteuerung der Einzelstrahler (3-1-;3-2) in diesem Verfahren berücksichtigt wird.

Das erfindungsgemäße Verfahren wird durch eine Rekonstruktionssoftware mit computerlesbarem Code implementiert, das von dem computergestützten Röntgengerät (2) ausgeführt werden kann.

Die Rekonstruktionssoftware ist auf einem computerlesbarem Speichermedium gespeichert.

Das computergestützte DVT-System (1) umfasst ein extraorales dentales Röntgengerät (2), und eine Recheneinheit (8), die zur Ausführung der Rekonstruktionssoftware konfiguriert ist.

Gemäß der vorliegenden Erfindung können die Datensätze, die durch die oben aufgeführte Ausführungsformen erzeugt werden, zur Visualisierung, insbesondere für diagnostische Zwecke, einem Arzt vorgelegt werden, vorzugsweise mittels der Anzeige (9) oder eines Ausdrucks.

### Kalibration

Es wird auch eine Kalibrierroutine bereitgestellt, welche das Strahlerarray (3) zusammen mit dem Röntgendetektor (4) kalibriert. Die Kalibrierroutine umfasst u.a. folgende Schritte:
- Kalibrierung der Dosisleistung der Einzelstrahler auf Basis des applizierten Stroms und der Spannung, und die Strahlzeit;
- Kalibrierung der örtlichen Strahlungsintensitätsverteilungen der Einzelstrahler auf der Detektorebene;
- Kalibrierung des Überlappungsbereichs der Einzelstrahler auf der Detektorebene;
- Verwendung (optional) eines geeigneten Prüfkörpers mit einem oder mehreren Materialien zur Kalibrierung der Einzelstrahler-spezifischen Röntgenspektren

## Patentansprüche

1. Extraorales dentales Röntgengerät (2) für Aufnahmen eines Patienten im dentalen Bereich umfassend:
ein Strahlerarray (3), welches mindestens zwei Einzelstrahlern (3-1;3-2) jeweils zum Emittieren von Röntgenstrahlungen aufweist, die mindestens entlang einer vorbestimmten Richtung (z) versetzt sind; und
einen Röntgendetektor (4) zum mindestens teilweisen Detektieren der von den Einzelstrahlern (3-1;3-2) emittierten Röntgenstrahlungen, wobei das Strahlerarray (3) und der Röntgendetektor (4) um eine zur der genannten vorbestimmten Richtung (z) parallel laufenden Achse (z') relativ zueinander beweglich gelagert sind;
**gekennzeichnet dadurch, dass** es ferner
eine Steuerungsvorrichtung zum separaten Steuern der Einzelstrahler (3-1;3-2) aufweist, wobei die Steuerungsvorrichtung so ausgebildet ist, dass die emittierten Röntgenstrahlungen der mindestens zwei Einzelstrahler (3-1;3-2) sich in der Intensität und/oder Spektralverteilung der Röntgenstrahlungen unterscheiden um eine entlang der vorbestimmten Richtung (z) variierbare Intensität und/oder Spektralverteilung zu bewirken.

2. Extraorales dentales Röntgengerät (2) nach Anspruch 1, **gekennzeichnet dadurch, dass** die Steuerungsvorrichtung so ausgebildet ist, dass die Einzelstrahler (3-1;3-2) sequenziell oder zeitgleich angesteuert werden können, und die detektierten Signale des Röntgendetektors (4) synchron zur Ansteuerung der Einzelstrahler (3-1;3-2) ausgelesen werden.

3. Extraorales dentales Röntgengerät (2) nach Anspruch 1 oder 2, **gekennzeichnet dadurch, dass** die Einzelstrahler (3-1;3-2) jeweils eine eigene Blendenvorrichtung aufweisen, oder das Strahlerarray (3) eine gemeinsame Blendenvorrichtung aufweist.

4. Extraorales dentales Röntgengerät (2) nach einem der vorangegangenen Ansprüche, **gekennzeichnet dadurch, dass** die Einzelstrahler (3-1;3-2) jeweils eine eigene Filtervorrichtung aufweisen, oder das Strahlerarray (3) eine gemeinsame Filtervorrichtung aufweist.

5. Extraorales dentales Röntgengerät (2) nach einem der vorangegangenen Ansprüche, **gekennzeichnet dadurch, dass** die Betriebsweise des Röntgengeräts (2) vom Benutzer mittels einer Eingabevorrichtung bzgl. mindestens einer der folgenden Konfigurationsgrößen wie PAN-, CEPH-, DVT- Bildaufnahmeverfahren, Zielwert für Intensität und/oder Spektralverteilung der Röntgenstrahlungen, Kollimierung durch Blendenvorrichtungen, Filtrierung durch Filtervorrichtung, Größe und/oder Lage des Aufnahmebereichs im Patientenkopf (10), Trajektorie des Strahlerarrays (3) und des Röntgendetektors (4), konfigurierbar ist, und die Steuerungsvorrichtung weiter ausgebildet ist die Einzelstrahler (3-1;3-2) auf Basis der Benutzerkonfiguration zu steuern.

6. Extraorales dentales Röntgengerät (2) nach einem der vorangegangenen Ansprüche, **gekennzeichnet dadurch, dass** die Steuerungsvorrichtung weiter ausgebildet ist die Einzelstrahler (3-1;3-2) für die Aufnahme so zu steuern, dass die Intensität und/oder Spektralverteilung der Röntgenstrahlungen adaptiv an die Anatomie des Patienten angepasst werden, wobei die Steuerungsvorrichtung die Anatomie des Patienten nach mindestens einer der folgenden Varianten ermittelt:
(a) basierend auf allgemeinen Annahmen zur menschlichen Anatomie,
(b) basierend auf der Einstellung einer Vorrichtung (6,7) zum Positionieren des Patienten;
(c) basierend auf der vorbekannten Anatomie des Patienten.

7. Extraorales dentales Röntgengerät (2) nach einem der vorangegangenen Ansprüche, **gekennzeichnet dadurch, dass** die Steuerungsvorrichtung weiter ausgebildet ist die Einzelstrahler (3-1;3-2) für die Aufnahme so zu steuern, dass die Intensität und/oder Spektralverteilung der Röntgenstrahlungen während der Aufnahme in Abhängigkeit vom Signal des Röntgendetektors (4) nachgeregelt werden.

8. Extraorales dentales Röntgengerät (2) nach einem der vorangegangenen Ansprüche, dass die Steuerungsvorrichtung weiter ausgebildet ist mindestens einen Einzelstrahler während der Aufnahme so zu steuern, dass die Intensität und/oder Spektralverteilung der Röntgenstrahlung des Einzelstrahlers (3-1;3-2) nach einer vorgegebenen Sequenz variiert werden, welche eine Frequenz von mindestens 50 Hz aufweist.

9. Extraorales dentales Röntgengerät (2) nach einem der vorangegangenen Ansprüche, **gekennzeichnet dadurch, dass** die Betriebsart des Röntgendetektors (4) für die jeweils verwendeten Intensität und/oder Spektralverteilung der Röntgenstrahlungen komplett oder bereichsweise vor oder während der Aufnahme angepasst wird, wobei die Betriebsart mindestens einer der folgenden Parameter umfasst: Dynamikbereich, Ausleserate, ausgelesener Bereich, und die Verstärkung.

10. Extraorales dentales Röntgengerät (2) nach einem der vorangegangenen Ansprüche, **gekennzeichnet dadurch, dass** das extraorale Röntgengerät (2) eines oder mehrere der PAN-, CEPH-, DVT- Bildaufnahmeverfahren aufweist, wobei diese durch den Benutzer wählbar sind.

11. Verfahren zur Berechnung eines gemeinsamen Bildes für PAN/CEPH oder eines Volumens für DVT aus Signalen des Röntgendetektors (4) des extraoralen dentalen Röntgengeräts (2) nach einem der vorangegangenen Ansprüche, wobei die Information der Ansteuerung der Einzelstrahler (3-1;3-2) im Verfahren berücksichtigt wird.

12. Die Verwendung eines Datensatzes zur Visualisierung der durch Anspruch 11 zur Verfügung gestellt wurde.

13. Rekonstruktionssoftware umfassend computerlesbaren Code, das wenn es von einem computergestützten DVT-System (1) ausgeführt wird dieses veranlasst, das Verfahren nach Anspruch 11 auszuführen.

14. Computerlesbares Speichermedium, das die Rekonstruktionssoftware nach Anspruch 13 speichert.

15. Computergestütztes DVT-System (1) umfassend ein extraorales dentales Röntgengerät (2) nach einem der Ansprüche 1 bis 10, und eine Recheneinheit (8), die zur Ausführung der Rekonstruktionssoftware nach Anspruch 13 konfiguriert ist.
